# EUROPEAN PATENT SPECIFICATION

(11) **EP 1 433 859 B1**
(45) Date of publication and mention of the grant of the patent: **29.11.2006**
(21) Application number: 02762979.9
(22) Date of filing: 03.09.2002
(51) Int. Cl.: C12Q 1/37, G01N 33/68, G01N 33/15, G01N 33/50, G01N 33/53, G01N 27/26

(54) **METHOD OF DETERMINING TARGET PROTEIN OF DRUG**
VERFAHREN ZUR BESTIMMUNG EINES ZIELPROTEINS EINES ARZNEISTOFFS
PROCEDE DE DETERMINATION D'UNE PROTEINE CIBLE D'UN MEDICAMENT

(30) Priority: 03.09.2001 JP 2001265732
(43) Date of publication of application: 30.06.2004
(73) Proprietor: KYOWA HAKKO KOGYO CO., LTD., Chiyoda-ku, Tokyo 100-8185 (JP)
(72) Inventor: SHARMA, Sreenath V., Sayre, PA 18840 (US); YOSHIDA, Chitose, c/o Tokyo Research Laboratories, Suita-shi, Tokyo 565-0821 (JP); NAKANO, Hirofumi, c/o Kyowa Hakko Kogyo Co.,Ltd., Machida-shi, Tokyo 194-8533 (JP)
(74) Representative: Harding, Charles Thomas
(86) International application number: PCT/JP2002/008923
(87) International publication number: WO 2003/020960

(56) References cited:
- EP-A- 0 770 876
- EP-A1- 0 770 876
- EP-A1- 1 085 097
- WO-A-98/59360
- US-A- 5 585 277
- SHIMIZU N ET AL: "High-performance affinity beads for identifying drug receptors" NATURE BIOTECHNOLOGY, NATURE PUBLISHING, US, vol. 18, August 2000 (2000-08), pages 877-881, XP002203018 ISSN: 1087-0156

## Description

### Technical Field

The present invention relates to a method for determining a target protein of a medicament.

### Background Art

A medicament exhibits its pharmacological effect on a living organism by binding to a specific substance in the living organism and changing a function of the substance. Mostly, the substance in the living organism is a protein, which is called as a target protein of a medicament. The functional change of a target protein which is induced by the binding of a medicament is considered to involve some structural changes of the target protein [US Patent Nos. 5585277 and 5679582, Japanese Published Unexamined Patent Application No. 9-178746, WO97/20952]. For the screening of novel medicaments, evaluation of medicaments discovered, and separation of side effects and the like, it is very important to determine a target protein of a medicament and study the functional change of the target protein when the target protein is bound with the medicament, and a correlation between the functional changes and the pharmacological effects, i.e., to elucidate a mechanism of action of the medicament for exhibiting its pharmacological effects in vivo.

In order to elucidate the mechanism of action of a medicament, it is essential to first determine a target protein. As methods for determination of a target protein of a medicament, known are a method which comprises purifying a target protein from tissues or cells by affinity chromatography using a property that a target protein binds to a medicament [Shimizu N et al., Nat. Biotechnol. 18, 877 (2000), Fruichi H et al., Biochem. Biophys. Res. Commun. 270, 1002 (2000), Jbilo O et al., J. Biol. Chem. 272, 27107 (1997)], and a method which comprises constructing a protein expression cDNA library, and isolating an expressing clone that binds to the medicament as a probe (Japanese Published Unexamined Patent Application No. 10-248571 and Japanese Published Unexamined Patent Application No. 2000-508923). By these methods, a target protein binding to a medicament or a gene encoding the target protein is first isolated, and then a target protein can be determined by analyzing its structure.

However, in these methods, a medicament and a carrier need to be conjugated. Further, for a determination of a target protein, the carrier should be designed so that an inherent stereostructure of the medicament is not destroyed, or the binding between the medicament and the target protein is not inhibited. Generally, a medicament is conjugated to a carrier by using a functional group which does not significantly influence the pharmacological activity of the medicament. However, in order to select a functional group which does not influence the activity, the structure-activity relationship data of the medicament is required. A problem thus arises that it takes much effort to obtain the structure-activity relationship data.

### Disclosure of the Invention

An object of the present invention is to provide a method for determining a target protein of a medicament. More specifically, an object of the present invention is to provide a method for easily and accurately determining a target protein of a medicament without conjugating the medicament to a carrier.

Generally, proteins, which naturally exist in cells, are known to have their inherent stereostructures with their polypeptide chains folded, and thus extremely stable [Levitt M et al., Annu. Rev. Biochem. 66, 549 (1997)]. The inventors of the present invention predicted that, when a target protein binds to a medicament to change its function, some structural changes may occur, thereby sensitivity to a protease is increased. In order to verify the hypothesis, the inventors of the present invention have administered a medicament to a cell, and analyzed changes in protease sensitivities of intracellular proteins. As a result, we found that a target protein changes into a protease-sensitive protein; and by a detection of a protein that shows a change in its protease sensitivity, the protein is determined as a target protein of said medicament. In a method of the present invention, a step of conjugating a medicament to a carrier can be omitted, and obtaining the structure-activity relationship data of a medicament is not required beforehand, and accordingly, a target protein of a medicament can be determined extremely easily and accurately. The present invention was achieved on the basis of these findings.

The present invention thus provides the following (1) to (13):
(1) A method for determining a target protein of a medicament, which comprises a step of judging that a protein which shows an increase in protease sensitivity in the presence of a medicament, is the target protein of said medicament.
(2) The method according to (1), which comprises a step of selecting a protein which shows an increase in protease sensitivity in the presence of a medicament, from among proteins contained in a cell lysate.
(3) The method according to (1) or (2), wherein an endogenous cellular protease is used as the protease.
(4) The method according to any one of (1) to (3), which further comprises a step of adding an exogenous protease.
(5) The method according to any one of (1) to (4), wherein the increase in the protease sensitivity is detected as an increase in a degradation amount of said protein.
(6) The method according to (5), which further comprises a step of detecting an inhibition of the increase of the degradation amount of said protein in the presence of a protease inhibitor.
(7) The method according to (5) or (6), wherein the change of the protein amount is detected by acrylamide gel electrophoresis.
(8) The method according to (7), wherein the acrylamide gel electrophoresis is SDS-polyacrylamide gel electrophoresis or two-dimensional electrophoresis.
(9) The method according to any one of (1) to (8), which comprises a step of identifying the selected protein.
(10) The method according to (9), which comprises a step of isolating and identifying the selected protein.
(11) The method according to any one of (1) to (8), which comprises a step of detecting said protein.
(12) The method according to (11), wherein the detection is conducted by using an antibody which recognizes said protein.
(13) The method according to (12), wherein the detection is conducted by Western blotting, dot blot, enzyme immunoassay, or radioimmunoassay.

### Brief Explanation of Drawings

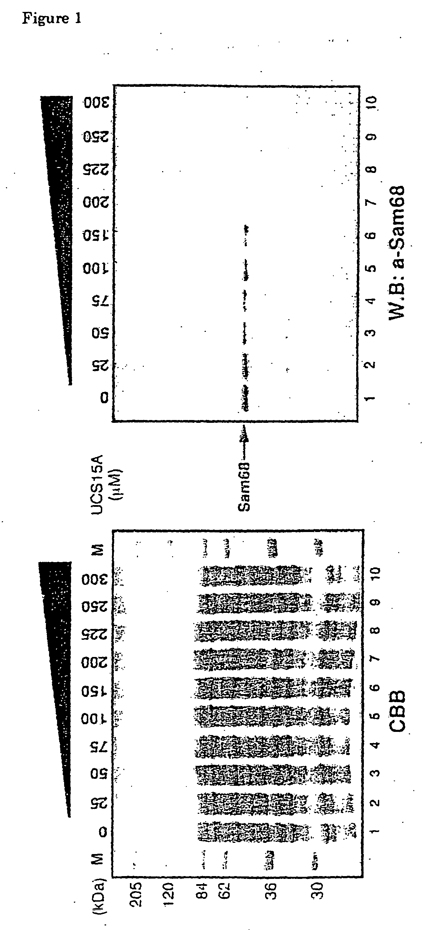

Fig. 1 shows a result of protease digestion assay of a protein by using a cell lysate of HCT116, wherein UCS15A was added at different concentrations. The left figure shows a result of Coomassie Brilliant Blue staining of a gel of SDS-PAGE, and the right figure shows a result of specific detection of Sam68 by Western blotting. In both of the figures, the number above each lane indicates a concentration (µmol/L) of UCS15A added to the cell lysate, M indicates molecular weight markers, and the numbers left side indicate molecular weights (kDa) of the molecular weight markers.

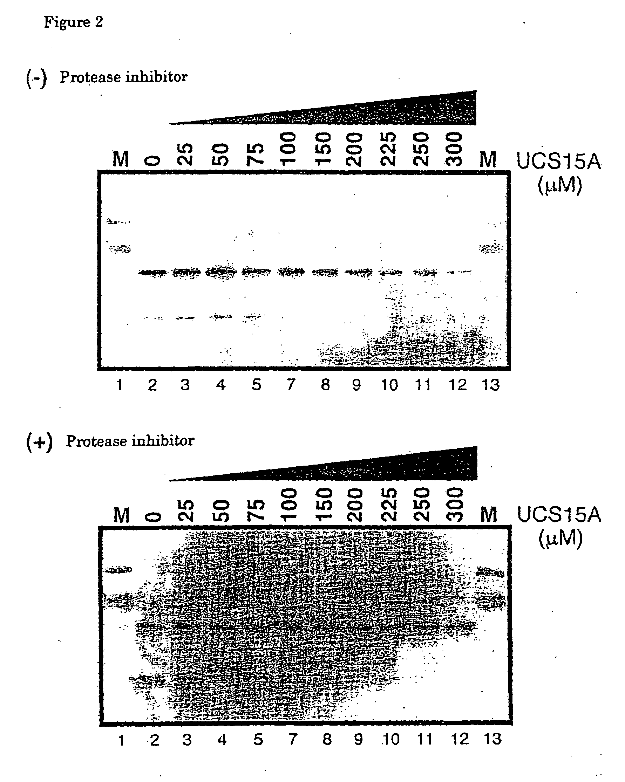

Fig. 2 shows an effect of a protease inhibitor on degradation of Sam68 in a cell lysate. The figure shows results of specific detection of Sam68 by Western blotting in a protease digestion assay wherein UCS15A was added at different concentrations: upper figure indicates the results without addition of a protease inhibitor, and the lower figure indicates the results with addition of a protease inhibitor. In both of the figures, the number above each lane indicates a concentration (µmol/L) of UCS15A added to the cell lysate, M indicates molecular weight markers.

### Best Mode for Carrying out the Invention

The method of the present invention is that for determining a target protein of a medicament, and characterized in that said method comprises a step of judging that a protein which shows an increase in protease sensitivity in the presence of a medicament, is the target protein of said medicament. The method of the present invention can be applied to a protein, which is predicted to be a target protein of a medicament, to judge whether or not said protein is the target protein of said medicament. In addition, the method of the present invention can be used to select a protein which shows an increase in protease sensitivity in the presence of a medicament, from a mixture of two or more proteins.

Preferably, the method of the present invention comprises a step of selecting a protein which shows an increase in protease sensitivity in the presence of a medicament, from among proteins contained in a cell lysate. By conducting the aforementioned selection by using a cell lysate in vitro, a target protein can be determined more easily than by analyzing in vivo, i.e., wherein a medicament is added during culturing cells; and changes of the protein derived only from direct actions of the medicament can be detected while excluding other changes of the protein caused by indirect actions such as secondary actions by signal transduction molecules downstream of a target protein and actions on synthesis or degradation mechanisms of the protein.

An increase in protease sensitivity of a protein in the presence of a medicament can generally be detected as an increase in a degradation amount of said protein. For example, a mixture of proteins is treated with a protease in the presence of a medicament to detect individual proteins, and a change in an amount of each protein can be analyzed. When a decrease in the amount of a protein is detected by the analysis, it is recognized that the protein is digested by the protease and the protease sensitivity of the protein is increased. For the foregoing detection, it is preferred that the same protease treatment is conducted in the absence of the medicament and the result is used as a control. As described above, a protein which shows an increase in protease sensitivity can be judged as the target protein of the medicament.

As the protease for carrying out the method of the present invention, a protease derived from a cell (sometimes referred to as "endogenous protease" in the specification) may be sufficient. If necessary, an appropriate type of protease (which is not derived from the cell; sometimes referred to as "exogenous protease" in the specification) may be further added thereto. Two or more types of proteases may be appropriately combined and added to the reaction system.

Further, the protease reaction may be conducted in the presence of a protease inhibitor, and by comparing the result with that of the reaction conducted in the absence of the protease inhibitor, it can be verified that, when a decrease in an amount of the protein is detected, the decrease is caused by an increase in a degradation amount of the protein by the protease, and not by the other causes such as aggregation of the protein.

As a preferred embodiment of the method of the present invention, a method wherein a cell lysate is used as a mixture of proteins will be specifically explained. However, the method of the present invention is not limited to the details of the following explanations.

### 1) Preparation of a cell lysate

A cell lysate can be prepared from cells or tissues for which a target protein of a medicament should be determined. As cells or tissues used for the preparation of a cell lysate, any materials such as an established cell-line, cells isolated from bloods and tissues, and tissues collected from a living organisms may be used. By using cell lines stored under a constant culture condition, and by applying the same conditions for preparations of cell lysates, qualitative changes in the total proteins contained in the cell lysate are reduced and reproducible results can be obtained.

A cell lysate can be prepared as a supernatant obtained by physically disrupting cells or tissues with a suitable buffer by using an ordinary homogenizer, for example, a homogenizer for tissues such as Dounce homogenizer and Polytron homogenizer, or by disrupting cells by a sonicator, and then centrifuging the resulting liquid. As the buffer, an approximately neutral phosphate-based buffer or a tris-based buffer which contains a salt such as NaCl at a low concentration, such as phosphate buffered saline (PBS) and reticulocyte swelling buffer (RSB: 10mmol/L Tris-hydrochloride pH 7.6, 10mmol/L NaCl, 1.5mmol/L MgCl₂), can be used.

### 2) Addition of a medicament

A medicament is added to the cell lysate obtained above. It is preferable that a cell lysate which is not added with the medicament is also prepared as a control. It is preferred to conduct experiments at plural concentrations of the medicament to observe that a protein degradation is dependent on the medicament concentration. When a medicament is solid, the medicament can be added after dissolving in the buffer used for the preparation of the cell lysate, or in any suitable solvent. For the above operation, it is preferred that the same solvent as that used for dissolving the medicament is added to the control cell lysate which is not added with the medicament. It is preferred that the volume of the solvent to be added is adjusted to the same volume among all the cell lysates.

### 3) Protease reaction

A protease is allowed to react in the above cell lysates added with the medicament. A cell lysate usually contains a sufficient amount of a protease derived from the cells. Therefore, the protease can be reacted by incubating a cell lysate itself at 25 to 42°C, preferably at 37°C. For verification that a decrease in an amount of a specific protein is caused by a protease digestion, it is preferred that a reaction added with a protease inhibitor, preferably a protease inhibitor mixture which extensively inhibits variety of proteases, is prepared, and the result is analyzed by comparing with that in the reaction wherein protease is allowed to react under the condition without the protease inhibitor. When a decrease in the protein amount is not observed in the reaction wherein a protease inhibitor is added, and a decrease in the protein amount is observed in the reaction wherein the protease inhibitor is not added, the decrease is considered to be caused by a protease digestion.

A period of time for treatment with a protease is not particularly limited. However, the period of time for the treatment should be suitably selected so as not to cause degradation of all of proteins in a cell lysate, and so as to be sufficient for a specific degradation of the target protein with an increased protease sensitivity. Since the present specification specifically describes a typical example of the method of the present invention using a cell lysate in detail examples, it goes without saying that those skilled in the art can appropriately choose the aforementioned period of time for the protease treatment depending on various conditions such as a type of cells and a type of a medicament by referring to the example of the present specification.

For example, cell lysates which are not added with medicaments are reacted with a protease by incubating the lysates in the same manner for different periods of time ranging from 30 minutes to 24 hours, and then proteins are detected by SDS-polyacrylamide gel electrophoresis. Among the applied period of the reaction time, a period of time can be chosen as the period of the reaction time which gives many clear bands of proteins, which are not smeared, in the region of more than 50 kDa, compared to a result before the treatment with the protease. Further, among the periods of time thus chosen, a longer period of time is preferred. Since amounts of contained proteases are different between cells, preferable periods of time for the treatment are different. For example, 6 to 12 hours is preferred for a HCT116 cell (ATCC number CCL-247) lysate.

When an exogenous protease is further added to the aforementioned cell lysate, examples of the protease include trypsin, chymotrypsin, V8 protease, elastase, carboxypeptidase, lysylendopeptidase, proteinase K, thermolysin, papain, subtilisin, and a mixture thereof.

### 4) Analysis of proteins and determination of a target protein

Every individual protein in the cell lysate treated with a protease under each condition, i.e., in the presence or absence of a protease inhibitor, is analyzed, and then an increase in protease sensitivity of each protein is detected. An increase in protease sensitivity of a protein usually corresponds to a decrease in an amount of the protein which is specifically observed in the analysis of proteins in the cell lysate under each of the conditions, which is detected as a decrease depending on the amount of a medicament, and also as a phenomenon of inhibition of the decrease in the presence of a protease inhibitor. By the above procedure, a protein which shows an increase in the protease sensitivity can be determined to be the target protein of the medicament. A method for identification of a target protein contained in a cell lysate, and a method for evaluation whether or not a certain specified protein is a target protein will be explained separately below.

### 4-1) Identification of a target protein

When a target protein is unknown, a protein which is detected an increase in protease sensitivity is isolated and subjected to a structural determination, thereby a target protein can be identified. For this procedure, it is generally preferred that total proteins in a cell lysate are analyzed. Examples of analysis methods include acrylamide gel electrophoresis such as SDS-polyacrylamide gel electrophoresis and two-dimensional electrophoresis. Gel after the electrophoresis, or a polyvinylidene difluoride (PVDF) membrane or a nitrocellulose membrane to which proteins in the gel are transferred, is subjected to non-specific staining such as Coomassie Brilliant Blue staining or silver staining, etc., to thereby analyze all of the proteins for detection.

As for the cell lysate added with a medicament, the detected patterns of total proteins, treated with a protease under each condition of the presence or absence of a protease inhibitor, are compared. A band or a spot of which the amount specifically decreases in the absence of a protease inhibitor depending on a medicament, and the decrease is inhibited in the presence of the protease inhibitor is chosen. A protein corresponding to the aforementioned band or spot has increased its protease sensitivity and become more susceptible to protease digestion as a result of a conformational change by binding to the medicament, and therefore, the protein can be determined to be the target protein of the added medicament.

In the gel or the membrane transferred with the proteins of the cell lysate which is not added with a medicament, the target protein, which gives a band or spot corresponding to the band or spot of which the amount decreases depending on the medicament in the above analysis, can be isolated and determined in its structure as described below. First, the corresponding band or spot is cut out, the protein is digested to peptides with a protease such as trypsin on the gel, and the peptide mixture is detected by using a MALDI-TOF (matrix assisted laser desorption ionization-time of flight) mass spectrometer. A theoretical mass pattern of the peptides calculated from a sequence on a sequence database with protease treatment and the experimentally obtained peptide sequence pattern.are compared. The protein, which gives a high MOWSE score [Pappin et al., Curr. Biol. 3, 327 (1993)] considering a concordance rate of mass patterns of peptide mixtures, can be determined to be the target protein.

Sometimes a target protein can not be determined by the above method, for example, when plural proteins are contained. For that mixture, analyses can be carried out by applying a tandem mass spectrometry using ESI (electro spray ionization) to obtain a partial sequence, and then the protein can be determined by searching a database of EST (expressed sequence tag) such as GenBank [Pandey A and Mann M, Nature 405, 837 (2000), Yaguchi, Experimental medical science 17, 2550 (1999)]. A protein in the database containing the corresponding amino acid sequence, which is found as a result of the searching by the method described above, can be determined to be the target protein. When the corresponding amino acid sequence is not found, the protein can be judged as a novel protein having an unknown amino acid sequence.

### 4-2) Evaluation whether or not a specified protein is a target protein of a medicament

When a certain specified protein is subjected to evaluation whether or not the protein is a target protein of a medicament, the objective protein for the evaluation is specifically detected instead of an analysis of all proteins. As a detection method specific for the protein, examples include methods using antibodies which can recognize the protein, i.e., Western blotting, dot blot, and enzyme immunoassay (EIA) such as sandwich ELISA, and radioimmunoassay (RIA). These methods can be conducted by referring to publications [Experimental manuals for monoclonal antibodies, edited by Sakuji Toyama and Tamie Ando, published by Kodansha Scientific (1987); Succeeding issue of lectures on biochemical experiments 5 "Methods for researches in immunological biochemistry, Tokyo Kagaku Dojin (1986); Goding JW, Monoclonal Antibodies: Principles and Practice, Third edition, Academic Press (1996); Harlow E and Lane D, Antibodies: A laboratory Manual, Cold Spring Harbor Laboratory (1988)].

When the amount of the protein decreases in the absence of a protease inhibitor depending on a medicament, and the decrease is inhibited in the presence of the protease inhibitor, the protein can be determined to be the target protein of the medicament, whereas when a decrease depending on a medicament is not observed, or when a decrease is observed also in the presence of the protease inhibitor, the protein is not considered to be the target protein of the medicament.

A preferred typical method of the present invention comprises the following steps (1) to (4):
(1) A cell lysate is prepared. Each of (a) a cell lysate added with a medicament (cell lysate (a)), and (b) a cell lysate added with the medicament and a protease inhibitor (cell lysate (b)) is prepared;
(2) The cell lysate (a), and the cell lysate (b) which are prepared in the above step (1), and a control cell lysate which is not added with a medicament or a protease inhibitor (control cell lysate) are incubated as they are, or together with an exogenous protease, thereby allowing the protease to react with proteins in the cell lysates,
(3) Every individual protein in each cell lysate is detected after incubating. A protein is selected which decreases its amount in the cell lysate (a) as compared with the control cell lysate, and the decrease of the amount is inhibited in the cell lysate (b).
(4) The selected protein (the target protein) is isolated and identified.
   Another typical method comprises the following steps (5) and (6) subsequent to the above steps (1) and (2).
(5) Every individual protein in each cell lysate after the incubation is separated by acrylamide gel electrophoresis and stained. A band or spot is selected which decreases its area in the cell lysate (a) as compared with the control cell lysate, and the decrease of the area is inhibited in the cell lysate (b).
(6) The protein forming the selected band or spot (the target protein) is isolated and identified.
   A further typical method comprises the following step (7) subsequent to the above steps (1) and (2).
(7) A certain specified protein in each cell lysate after the incubation is detected. It is confirmed that an amount of said protein is decreased in the cell lysate (a) as compared with the control cell lysate, and the decrease of the amount is inhibited in the cell lysate (b).

### Examples

The present invention will be more specifically explained with reference to the following examples. However, the scope of the present invention is not limited to these examples.

UCS15A was used as a medicament in the following example. This medicament is isolated from a culture broth of *Streptmyces actinobacterium*, and has an inhibitory activity on bone resorption (Japanese Published Unexamined Patent Application No. 8-268888). The medicament is identical to the substance reported as SI-4228 in Japanese Published Unexamined Patent Application No. 62-28959. UCS15A has, as well as the inhibitory activity on bone resorption, various activities such as a bactericidal activity (Japanese Published Unexamined Patent Application Nos. 58-116686 and 63-22583), an immunosuppressive activity (Japanese Published Unexamined Patent Application No. 61-293920), and an antitumor activity (Japanese Published Unexamined Patent Application No. 63-48213). However, action mechanisms and target proteins have been remained unknown. Recently, UCS15A is reported to inhibit the signal transduction of tyrosine kinase src, whereas not to inhibit the enzyme activity of src, per se. The compound is also reported to specifically inhibit the binding between src and Sam68 which is known as the substrate of src [Sharma SV et al., Oncogene, 20, 2068 (2001)], which strongly suggests that one of target proteins of UCS15A is Sam68.

### (1) Preparation of a cell lysate

By using Dulbecco's modification of Eagle's medium (DMEM) containing 10% fetal bovine serum, human colon tumor cell line HCT116 (ATCC number CCL-247) was cultured on 100 mm diameter dishes for cell culturing in a CO₂ incubator at 37°C under 5 % CO₂ condition, and proliferated by subculture with division into five portions every 3 days. The culture medium was removed from three HCT116 cell dishes on the third day from the subculture, and cells were washed by addition and successive removal of 10 ml of ice-cooled PBS. To these cells, 10mL of RSB (10mmol/L Tris-hydrochloride pH7.6, 10mmol/L NaCl, 1.5mmol/L MgCl₂) having a low osmotic pressure was added. After the cells were put on ice for 10 minutes to allow expansion, the cells were detached from the dishes and put into 15 mL volume Dounce homogenizer together with the already added RBS. The cells were disrupted by 50 strokes of a pestle. This cell lysate was centrifuged at 4°C, 15,000 rpm for 30 minutes. The obtained supernatant was collected, and divided into ten 1 mL aliquots.

### (2) Protease digestion assay of UCS15A

UCS15A was isolated and purified from *Streptmyces actinobacterium*, according to the descriptions in Japanese Published Unexamined Patent Application No. 58-116686, and a dimethylsulfoxide (DMSO) solution at a concentration of 10 mmol/L was prepared. UCS15A was added to each of the 10 cell lysates of HCT116 prepared in (1) to obtain the final concentrations of 0 (DMSO alone), 25, 50, 75, 100, 150, 200, 225, 250, and 300 µ mol/L. The added UCS15A was diluted with DMSO beforehand, so that the same volume of DMSO was added in the suspensions.

The cell lysates added with UCS15A was incubated at 37 °C for 12 hours to allow reaction of an cellular endogenous protease while the lysate was rotated gently by using a rotating incubator. To each of the cell lysates after the reaction was added 333 µL of 4 x concentration of Laemmli's sample buffer. After sufficient mixing, the mixtures were heated at 100°C for 10 minutes. Each 20 µL of the solutions was subjected to 8.5 % polyacrylamide gel electrophoresis. The gels after the electrophoresis were stained with Coomassie Brilliant Blue (Nacalai Tesque). The results are shown in figure 1 (the left figure). Several proteins, which decreased their amounts with the increase of UCS15A concentration, were observed.

### (3) Detection of Sam68 by western blotting

Sam68 in the cell lysate was detected by Western blotting as described below. Proteins in the gel after the electrophoresis were blotted to a nitrocellulose membrane with pore diameters of 0.45 µm (Protran; manufactured by Schleicher and Schuell). PBS containing 0.25% gelatin and 0.2% Tween-20 (hereinafter called as "PBS-TG") was loaded on the membrane and left overnight at 4°C, to block non-specific bindings. Then, as a primary antibody, rabbit anti-Sam68 polyclonal antibody (manufactured by Santa Cruz) diluted by 1:1000 with PBS-TG was reacted for 2 hours. After washing with PBS containing 0.2% Tween-20 (hereinafter called as "PBS-T"), horse radish peroxidase (HRP)-conjugated goat anti-rabbit IgG antibody (manufactured by Amersham Pharmacia Biotech), as secondary antibody diluted by 1:4000 with PBS-TG, was reacted for one hour. The detection was conducted by chemiluminescence using ECL reagent (manufactured by Amersham Pharmacia Biotech). As a result, as shown in figure 1 (the right figure), it was observed that the amount of Sam68 in the cell lysate decreased with the increase of the UCS15A concentration.

### (4) Effect of protease inhibitor

Whether the decrease of the Sam68 amount in (3) was due to a protease digestion was verified by the addition of a protease inhibitor as described below.

When the protease digestion assay described in (2) was conducted, a protease inhibitor cocktail (Complete; manufactured by Roche) which can inhibit wide variety of proteases was added along with UCS15A, and the same reaction was proceeded. After the electrophoresis, Sam68 was detected by Western blotting in a same manner to that of (3). A condition for the addition of the inhibitor and other conditions were in accordance with the manufacturer's manual attached to the reagent. As a result, as shown in figure 2, the decrease of the amount of Sam68 in the cell lysate was inhibited by the addition of a protease inhibitor. Therefore, it was verified that the decrease of the Sam68 amount is due to protease digestions, and that Sam68 becomes sensitive to an endogenous protease in the presence of UCS15A.

In the Western blotting in the presence or absence of the protease inhibitor shown in figure 2, the same amounts of the cell lysates were used, and periods of time for exposure for detection were the same. However, when each lane 2 in both results were compared, it was found that, without the addition of UCS15A, the Sam68 protein amount in the absence of a protease inhibitor was not different from that in the presence of a protease inhibitor, even when they were incubated at 37°C for 12 hours. Therefore, Sam68 was demonstrated to be inherently very stable to the action of the exogenous protease.

### Industrial Applicability

According to the method of the present invention, a target protein of a medicament can be easily and accurately determined. The method is extremely useful as means for development of medicaments such as screening of novel medicaments, evaluation of medicaments discovered, and separation of side effects.

## Claims

1. A method for determining a target protein of a medicament, which comprises a step of judging that a protein which shows an increase in protease sensitivity in the presence of a medicament, is the target protein of said medicament.

2. The method according to claim 1, which comprises a step of selecting a protein which shows an increase in protease sensitivity in the presence of a medicament, from among proteins contained in a cell lysate.

3. The method according to claim 1 or 2, wherein an endogenous intracellular protease is used as the protease.

4. The method according to any one of claims 1 to 3, which further comprises a step of adding an exogenous protease.

5. The method according to any one of claims 1 to 4, wherein the increase in the protease sensitivity is detected as an increase in a degradation amount of said protein.

6. The method according to claim 5, which further comprises a step of detecting an inhibition of the increase in the degradation amount of said protein in the presence of a protease inhibitor.

7. The method according to claim 5 or 6, wherein the change of the protein amount is detected by acrylamide gel electrophoresis.

8. The method according to claim 7, wherein the acrylamide gel electrophoresis is SDS-polyacrylamide gel electrophoresis or two-dimensional electrophoresis.

9. The method according to any one of claims 1 to 8, which comprises a step of identifying the selected protein.

10. The method according to claim 9, which comprises a step of isolating and identifying the selected protein.

11. The method according to any one of claims 1 to 8, which comprises a step of detecting said protein.

12. The method according to claim 11, wherein the detection is conducted by using an antibody which recognizes said protein.

13. The method according to claim 12, wherein the detection is conducted by Western blotting, dot blot, enzyme immunoassay, or radioimmunoassay.

## Patentansprüche

1. Verfahren zur Bestimmung eines Zielproteins eines Medikaments, welches die Stufe umfaßt, bei der man beurteilt, ob ein Protein, welches in Gegenwart eines Medikaments eine Steigerung der Proteaseempfindlichkeit zeigt, das Zielprotein des Medikaments ist.

2. Verfahren nach Anspruch 1, welches die Stufe umfaßt, bei der man ein Protein, welches in Gegenwart eines Medikaments eine Steigerung der Proteaseempfindlichkeit zeigt, unter in einem Zelllysat enthaltenen Proteinen auswählt.

3. Verfahren nach Anspruch 1 oder 2, wobei eine endogene intrazelluläre Protease als die Protease verwendet wird.

4. Verfahren nach einem der Ansprüche 1 bis 3, welches weiterhin die Stufe umfaßt, bei der man eine exogene Protease zugibt.

5. Verfahren nach einem der Ansprüche 1 bis 4, wobei die Steigerung der Proteaseempfindlichkeit als eine Steigerung der Abbaumenge des Proteins detektiert wird.

6. Verfahren nach Anspruch 5, welches weiterhin eine Stufe umfaßt, bei der man die Inhibition der Steigerung der Abbaumenge des Proteins in Gegenwart eines Proteaseinhibitors detektiert.

7. Verfahren nach Anspruch 5 oder 6, wobei die Veränderung der Proteinmenge mittels Acrylamidgelelektroforese detektiert wird.

8. Verfahren nach Anspruch 7, wobei die Acrylamidgelelektroforese SDS-Polyacrylamidgelelektroforese oder zweidimensionale Elektroforese ist.

9. Verfahren nach einem der Ansprüche 1 bis 8, welches die Stufe umfaßt, bei der man das ausgewählte Protein identifiziert.

10. Verfahren nach Anspruch 9, welches die Stufe umfaßt, bei der man das ausgewählte Protein isoliert und identifiziert.

11. Verfahren nach einem der Ansprüche 1 bis 8, welches die Stufe umfaßt, bei der man das Protein detektiert.

12. Verfahren nach Anspruch 11, wobei die Detektion unter Verwendung eines das Protein erkennenden Antikörpers durchgeführt wird.

13. Verfahren nach Anspruch 12, wobei die Detektion mittels Western-Blot, Dot-Blot, Enzymimmuntest oder Radioimmuntest durchgeführt wird.

## Revendications

1. Méthode pour la détermination d'une protéine cible d'un médicament, qui comprend une étape consistant à juger qu'une protéine qui présente une augmentation de sensibilité aux protéases en présence d'un médicament est la protéine cible dudit médicament.

2. Méthode suivant la revendication 1, qui comprend une étape de sélection d'une protéine qui présente une augmentation de sensibilité aux protéases en présence d'un médicament, parmi les protéines présentes dans un lysat cellulaire.

3. Méthode suivant la revendication 1 ou 2, dans laquelle une protéase intracellulaire endogène est utilisée comme protéase.

4. Méthode suivant l'une quelconque des revendications 1 à 3, qui comprend en outre une étape d'addition d'une protéase exogène.

5. Méthode suivant l'une quelconque des revendications 1 à 4, dans laquelle l'augmentation de la sensibilité aux protéases est détectée par une augmentation de la quantité dégradée de ladite protéine.

6. Méthode suivant la revendication 5, qui comprend en outre une étape de détection d'une inhibition de l'augmentation de la quantité dégradée de ladite protéine en présence d'un inhibiteur de protéases.

7. Méthode suivant la revendication 5 ou 6, dans laquelle la variation de la quantité de protéine est détectée par électrophorèse sur gel d'acrylamide.

8. Méthode suivant la revendication 7, dans laquelle l'électrophorèse sur gel d'acrylamide est l'électrophorèse sur gel de polyacrylamide-SDS ou l'électrophorèse bidimensionnelle.

9. Méthode suivant l'une quelconque des revendications 1 à 8, qui comprend une étape d'identification de la protéine sélectionnée.

10. Méthode suivant la revendication 9, qui comprend une étape d'isolement et d'identification de la protéine sélectionnée.

11. Méthode suivant l'une quelconque des revendications 1 à 8, qui comprend une étape de détection de ladite protéine.

12. Méthode suivant la revendication 11, dans laquelle la détection est effectuée en utilisant un anticorps qui reconnaît ladite protéine.

13. Méthode suivant la revendication 12, dans laquelle la détection est effectuée par transfert d'empreinte par la méthode Western, transfert d'empreinte dot blot, analyse immuno-enzymatique ou analyse radio-immunologique.
